# EUROPÄISCHE PATENTSCHRIFT

(11) **EP 1 248 948 B1**
(45) Veröffentlichungstag und Bekanntmachung des Hinweises auf die Patenterteilung: **13.10.2004**
(21) Anmeldenummer: 01915154.7
(22) Anmeldetag: 22.01.2001
(51) Int. Cl.: G01N 21/64, G01N 21/84, G01N 33/543, G01N 33/58, G01N 33/68, C12Q 1/68

(54) **VERFAHREN UND VORRICHTUNG ZUR BESTIMMUNG TEMPERATURABHÄNGIGER PARAMETER UND/ODER DER GLEICHGEWICHTSKONSTANTE VON KOMPLEXEN, DIE ZUMINDEST ZWEI KOMPONENTEN UMFASSEN**
METHOD AND DEVICE FOR DETECTING TEMPERATURE-DEPENDENT PARAMETERS, SUCH AS ASSOCIATION/DISSOCIATION PARAMETERS AND/OR THE EQUILIBRIUM CONSTANT OF COMPLEXES THAT COMPRISE AT LEAST TWO COMPONENTS
PROCEDE ET DISPOSITIF POUR LA DETERMINATION DE PARAMETRES DEPENDANT DE LA TEMPERATURE, TELS QUE LES PARAMETRES D'ASSOCIATION/DISSOCIATION ET/OU LA CONSTANTE D'EQUILIBRE DE COMPLEXES CONSTITUES D'AU MOINS DEUX COMPOSANTS

(30) Priorität: 21.01.2000 DE 10002566
(43) Veröffentlichungstag der Anmeldung: 16.10.2002
(73) Patentinhaber: Fraunhofer-Gesellschaft zur Förderung der angewandten Forschung e.V., 80686 München (DE)
(72) Erfinder: BRANDENBURG, Albrecht, 79232 March (DE); LEHR, Hans-Peter, 79110 Freiburg (DE); KLAPPROTH, Holger, 79108 Freiburg (DE); REIMANN-ZAWADZKI, Meike, 66119 Saarbruecken (DE)
(74) Vertreter: Zimmer, Franz-Josef, Dr.
(86) Internationale Anmeldenummer: PCT/EP2001/000664
(87) Internationale Veröffentlichungsnummer: WO 2001/053822

(56) Entgegenhaltungen:
- EP-A- 0 245 206
- EP-A- 0 892 070
- WO-A-96/35940
- WO-A-99/61148
- US-A- 5 599 668
- US-A- 5 677 196
- STIMPSON D I ET AL: "The utility of optical waveguide DNA array hybridization and melting for rapid resolution of mismatches, and for detection of minor mutant components in the presence of a majority of wild type sequence: Statistical model and supporting data" GENETIC ANALYSIS: BIOMOLECULAR ENGINEERING, ELSEVIER SCIENCE PUBLISHING, US, Bd. 13, Nr. 3, 1. September 1996 (1996-09-01), Seiten 73-80, XP004070186 ISSN: 1050-3862
- COLEMAN R S ET AL: "Thionucleoside Disulfides as Covalent Constraints of DNA Conformation" TETRAHEDRON, ELSEVIER SCIENCE PUBLISHERS, AMSTERDAM, NL, Bd. 55, Nr. 41, 8. Oktober 1999 (1999-10-08), Seiten 12009-12022, XP004178972 ISSN: 0040-4020

### SEQUENZPROTOKOLL

<110> Fraunhofer-Gesellschaft zur Förderung der angewandten Forschung e.V. et al.
<120> Verfahren und Einrichtung zur Bestimmung der Assoziations-/Dissoziationsparameter und/oder der Gleichgewichtskonstante von aus mindestens zwei Komponenten gebildeten Komplexen als Funktion der Temperatur
<130> PCT1310-031
<140>
   <141>
<150> DE 100 02 566.8
   <151> 2000-01-21
<160> 2
<170> PatentIn Ver. 2.1
<210> 1
   <211> 17
   <212> DNA
   <213> Künstliche Sequenz
<220>
   <223> Beschreibung der künstlichen Sequenz: artificial sequence
<400> 1
   atatacgtgc caggtgg                                          17
<210> 2
   <211> 17
   <212> DNA
   <213> Künstliche Sequenz
<220>
   <223> Beschreibung der künstlichen Sequenz: artificial sequence
<400> 2
   atatacgtac caggtgg                                          17

## Beschreibung

Die Erfindung betrifft ein Verfahren und eine Einrichtung zur Bestimmung temperaturabhängiger Parameter, wie der Assoziations-/Dissoziationsparameter und/oder der Gleichgewichtskonstante von aus mindestens zwei Komponenten gebildeten Komplexen, wobei die in einer Flüssigphase befindlichen ersten Komponenten mit einer Vielzahl von Meßpunkten auf einem optisch anregbaren Reaktionsträger, gebildet durch an den festen Reaktionsträger gekoppelte spezifisch an die ersten Komponenten bindende zweite Komponenten, in Kontakt gebracht werden und unter Einstrahlung von Anregungslicht, insbesondere Laserlicht, ein Fluoreszenzlicht erzeugt wird, das über eine Erfassungseinrichtung ausgewertet wird.

In biologischen und chemischen Systemen ist die Bildung (Assoziation) und der Zerfall (Dissoziation) von Komplexen relevant. Beispielsweise wird der Blutzuckerspiegel durch die Bindung von Insulin an seinen zellulären Rezeptor, d.h. durch die Bildung eines Insulin/Rezeptor-Komplexes gesteuert. Der gebildete Komplex führt hierbei zu einer Senkung des Blutzuckerspiegels. Weitere Beispiele für Komplexe in biologischen Systemen sind z.B. Antigen/Antikörper- und Enzym/Substrat-Komplexe.

Für die biologische und chemische Aktivität des Komplexes sind hierbei sowohl die kinetischen Parameter, d.h. die Assoziations- als auch Dissoziationskonstante, wie auch die thermodynamischen Parameter, d.h. die Gleichgewichtskonstante relevant. Die Temperaturabhängigkeit der vorgenannten Größen ist bekannt.

Die natürlich vorkommende Desoyribonukleinsäure (DNA) liegt allgemein als Doppelstrang, d.h. als Komplex aus zwei komplementären Nukleinsäure-Einzelsträngen vor. Die Geschwindigkeit von Replikations- und Transkriptionsprozessen hängt hierbei stark von der Verteilung zwischen Komplex und Einzelsträngen ab.

Das Dissoziieren des Komplexes in zwei voneinander getrennte Einzelstränge wird allgemein als "Schmelzen" und die Temperatur, bei der ca. 50 % des Komplexes in die getrennten Einzelstränge dissoziiert sind, als "Schmelztemperatur" bezeichnet. Allgemein nimmt die Tendenz zum Schmelzen des Komplexes bei zunehmender Temperatur zu..

Zur Bestimmung von Substanzen in Proben, insbesondere zur Feststellung bestimmter DNA-Sequenzen in einer Probe, ist die Verwendung von Biochips bekannt. Diese bilden planare Substanzträger, auf deren Oberfläche eine Vielzahl von Meßpunkten, z.B. gebildet durch Nukleinsären (komplementäre DNA-Einzelstränge) immobilisert sind, wobei diese Chipoberfläche mit einer die DNA-Sequenzen als zu analysierende Substanzen enthaltenden Probe in Kontakt gebracht werden und die Probe die zu analysierenden Nukleinsäuren enthält. Da jeder Einzelstrang eines Nukleinsäure-Moleküls mit seinem komplementären Strang eine Bindung eingeht, die als Hybridisierung bezeichnet wird, erhält man nach Prüfung der einzelnen Meßpunkte hinsichtlich der Anbindung von Probenmolekülen eine Aussage über die in der Probe vorhandenen DNA-Sequenzen. Einer der Vorteile der Biochip-Analytik besteht darin, daß bis zu einigen tausend Hybridisierungsereignissen parallel auf einem Biochip durchgeführt und detektiert werden können.

Entsprechend der Parallelität der Hybridisierungsereignisse ist ein Analysator zur Auswertung des Biochips erforderlich, der bei hoher Ortsauflösung eine ebenfalls hohe Nachweisempfindlichkeit erreicht. Da ein möglichst geringer Aufwand bei der Probenvorbehandlung getrieben werden soll, muß außerdem auch eine geringe Anzahl von hybridisierten Molekülen an den einzelnen Meßpunkten noch zuverlässig erkannt werden.

Heute kommerziell erhältliche Biochip-Reader arbeiten nach dem Scanning-Prinzip. Das Licht zur Anregung der Fluoreszenz tastet die Oberfläche seriell ab. Entweder wird bei feststehendem Lichtstrahl der Biochip schnell bewegt, oder es wird, zumindest für eine Bewegungsrichtung, ein Galvano-Scanner eingesetzt, mit dem der Lichtstrahl abgelenkt wird. Das von den Fluorochromen emittierte Licht wird dann mit einem empfindlichen Fotodetektor (z.B. einem Foto-Multiplier) nachgewiesen. Die Geräte sind als Labormeßsysteme für Anwendungen in der molekularbiologischen Forschung ausgelegt. Das Detektionslimit liegt im Bereich von wenigen Molekülen pro µm² bis ca. 100 pro µm². Die Scannzeiten liegen bei ca. 2 bis 4 Minuten. Die Kosten für derartige Geräte bewegen sich von ca. 50.000 US-$ für ein preisgünstiges Gerät bis zu ca. 350.000 US-$ für hochwertigere Geräte.

Beispielhaft werden hier die beiden Geräte genauer diskutiert, die heute vermutlich die größte Verbreitung haben. Es handelt sich hierbei um den GeneArray Scanner von Hewlett-Packard, der von der amerikanischen Firma Affymetrix vertrieben wird, und um den ScanArray 3000 von GSI Lumonics. Der GeneArray Scanner tastet die Chip-oberfläche optisch ab, indem in einer Raumrichtung eine Schnellablenkung des Lichtstrahles erfolgt. In der anderen Richtung wird der Chip schrittweise bewegt. Die Abmessungen des Gerätes betragen 66 cm x 78 cm x 42 cm. Die Lichtquelle ist ein Argon-Ionenlaser (Wellenlänge 488 nm). Detektiert wird mit einem Foto-Multiplier bei 550 bis 600 nm. Der ScanArray 3000 hat eine feststehende Optik. Der Scann-Vorgang wird durch eine schnelle Bewegung des Chips in einer Raumrichtung und eine schrittweise Verschiebung in der anderen Richtung realisiert. Es werden bis zu drei verschiedene Anregungswellenlängen zur Anregung verschiedener Fluorochrome angeboten. Die Detektion erfolgt auch hier mit einem Foto-Multiplier. Alle Meßgeräte werten den Biochip nach abgeschlossener Hybridisierung aus.

All diesen Geräten ist aber gemein, dass sie die Temperaturabhängigkeit relevanter kinetischer und thermodynamischer Parameter, wie z.B. der Assoziations- und Dissoziationskonstanten als auch der Gleichgewichtskonstante nicht zu erfassen vermögen.

Diese Geräte weisen ferner Probleme bei der parallelen Messung des Schmelzpunktes eines Nukleinsäurehybrides auf, von dem ein Strang an einer Festphase immobilisiert ist. Der Schmelzpunkt teilweise komplementärer Nukleinsäuren läßt sich mathematisch nur sehr ungenau errechnen, insbesondere, wenn festphasengebundene Reaktionspartner die Freiheitsgrade der Reaktion einschränken.

Eine weitere, diesen Geräten innewohnende Problematik besteht in der Bestimmung der Hybridisierungskinetik komplexer Proben zur Analyse und Konzentrationsbestimmung mehrerer Nukleinsäuren in einem Analysat. Der Nachweis multipler Nukleinsäuren durch Hybridisierung wird durch die Schmelzpunktproblematik der Hybride limitiert. Liegen die wirklichen, von den errechneten Schmelzpunkten oft abweichenden Schmelzpunkte der Hybride nicht in einem engen Temperaturbereich, so kann eine Messung dadurch sowohl qualitativ, also falsch-negativ, bzw. falsch- positiv sein, als auch im quantitativen Bereich gestört werden.

EP-A-0 245 206 offenbart ein Verfahren und eine Vorrichtung zur Bestimmung der Schmelztemperatur von einer an einer Lichtwellenleiteroberfläche gebundenen doppelsträngigen DNA, die mit einem fluoreszierenden Farbstoff markiert ist. Der Reaktionsträger umfaßt ein einzelner Meßpunkt. D. I. Stimpson & J. Gordon (1996) Gen. Anal. Biomol. Engineer. 13, 73-80 und US-A-5,599,668 offenbaren ein Verfahren und eine Vorrichtung zur Untersuchung der temperaturabhängigen Assoziations-/Dissoziationsreaktionen von doppelsträngigen DNA-Komplexen. Einzelsträngige Oligonukleotide werden als eine Vielzahl von Meßpunkten auf der Oberfläche eines Lichtwellenleiters immobilisiert und mit (nicht fluoreszierenden) markierten Oligonukeotiden hybridisiert. Das Anregungslicht wird durch die Markierungen gestreut, wobei das gestreute Licht mittels einer Abbildungsoptik erfaßt wird.

Der Erfindung liegt daher die Aufgabe zugrunde, ein Verfahren und eine Einrichtung der eingangs genannten Art derart zu verbessern, daß in einfacher Weise und ohne hohe Anforderungen an die Probenvorbehandlung eine präzise Bestimmung temperaturabhängiger Parameter, wie der Assoziations-/Dissoziationsparameter und/oder der Gleichgewichtskonstante ermöglicht wird.

Diese Aufgabe wird erfindungsgemäß gelöst durch ein Verfahren zur Bestimmung temperaturabhängiger Parameter, wie der Assoziations-/Dissoziationsparameter und/oder der Gleichgewichtskonstante von aus mindestens zwei Komponenten gebildeten Komplexen, wobei die in einer Flüssigphase befindlichen ersten Komponenten mit einer Vielzahl von Meßpunkten auf einem optisch anregbaren Reaktionsträger, gebildet durch an den festen Reaktionsträger gekoppelte, spezifisch an die ersten Komponenten bindende zweite Komponenten in Kontakt gebracht werden durch Inkontaktbringen der Flüssigphase und des Reaktionsträgers unter Bildung von Komplexen und die Anregung von an die ersten Komponenten und/oder zweiten Komponenten gebundenen, fluoreszierenden oberflächennahenFarbstoffen zur Emission von Fluoreszenzlicht durch eingestrahltes Anregungslicht und Detektion des emittierten Fluoreszenzlichtes in einem variablen Temperaturfeld erfolgt und die Bildung oder der Zerfall der Komplexe umfassend erste Komponenten und zweite Komponenten als Funktion der Temperatur beobachtet wird.

In einer bevorzugten Ausführungsform des erfindungsgemäßen Verfahrens sind die ersten und/oder zweiten Komponenten Rezeptoren und/oder Liganden.

In diesem Zusammenhang bedeutet der Ausdruck "Ligand" ein Molekül, das an einen bestimmten Rezeptor bindet. Liganden umfassen unter anderem Agonisten und Antagonisten für Zellmembranrezeptoren, Toxine, biologische Giftstoffe, virale Epitope, Hormone (z.B. Opiate, Steroide), Hormonrezeptoren, Peptide, Enzyme, Enzymsubstrate, Cofaktoren, Arzneistoffe, Lektine, Zucker, Oligonukleotide, Nukleinsäuren, Oligosaccharide, Proteine und Antikörper.

In diesem Zusammenhang bedeutet der Ausdruck "Rezeptor" ein Molekül, das eine Affinität für einen Liganden aufweist. Rezeptoren können natürlich vorkommende oder synthetisch hergestellte Rezeptoren sein. Rezeptoren können als Monomer oder als Heteromultimer in Aggregat-Form zusammen mit anderen Rezeptoren verwendet werden. Beispielhafte Rezeptoren umfassen Agonisten und Antagonisten für Zellmembranrezeptoren, Toxine, biologische Giftstoffe, virale Epitope, Hormone (z.B. Opiate, Steroide), Hormonrezeptoren, Peptide, Enzyme, Enzymsubstrate, Cofaktoren, Arzneistoffe, Lektine, Zucker, Oligonukleotide, Nukleinsäuren, Oligosaccharide, Zellen, Zell-Fragmente, Gewebe-Fragmente, Proteine und Antikörper.

Die Liganden und/oder Rezeptoren können kovalent oder nicht-kovalent mit dem Reaktionsträger verbunden sein. Die Bindung kann hierbei in dem Fachmann bekannter Weise erfolgen.

In einer weiteren bevorzugten Ausführungsform sind die ersten und/oder zweiten Komponenten Nukleinsäure-Einzelstränge. Besonders bevorzugt sind die Nukleinsäure-Einzelstränge wenigstens teilweise komplementär zueinander. Die wenigstens teilweise komplementären Nukleinsäure-Einzelstränge bilden unter geeigneten Bedingungen ein Nukleinsäurehybrid. Sowohl die temperaturabhängige Bildung als auch der Zerfall (Schmelzen) des Nukleinsäurehybrides können durch das erfindungsgemäße Verfahren bestimmt werden.

Durch die exakte Kenntnis der Schmelzpunkte von Nukleinsäure-Komplexen ist es möglich, die Mutationsanalyse auf Biochips wesentlich zu verbessern. Somit können Meßdaten für ein rationales Sondendesign zur Nukleinsäureanalytik entwickelt werden. Insbesondere ist es dadurch möglich, Sonden zur parallelen isothermischen Analyse zu verwenden.

Dabei ist es von Vorteil, wenn das Anregungslicht in den Lichtwellenleitern mittels einer optischen Einrichtung, wie z.B. einem Prisma, eingekoppelt wird.

Ferner kann das Anregungslicht durch Totalreflexion (ATR) oder Totale Interne Reflexions Fluoreszenz (TIRF) der Lichtstrahlen an einer Grenzfläche zwischen zwei optisch unterschiedlich dichten Medien im optisch dünneren Medium ein elektromagnetisches Feld erzeugen, wobei das optisch dichtere Medium eine Festphase, und das optisch dünnere Medium eine Flüssigphase ist, zur Messung des zeitlichen Verlaufs der Reaktion.

Ein solches Verfahren ermöglicht eine sehr einfache Auslegung des Reaktionsträgers, (Biochip) vorzugsweise durch Beschichten eines transparenten Körpers mit einer hochbrechenden planaren Wellenleiterschicht und gestattet eine einfache Analysevorrichtung, bei der z.B. eine die Probe enthaltende Flußzelle (oder einer Küvette oder sonstiger stationärer Probenkörper) in abdichtenden Oberflächenkontakt mit dem Reaktionsträger, insbesondere Biochip, gebracht wird, dessen Oberfläche zumindest im wesentlichen als planarer Wellenleiter ausgebildet ist und das Anregungslicht, das an einem Ende des planaren Wellenleiters in diesen eingekoppelt wird, führt, wobei das vom evaneszenten Feld des Anregungslichtes angeregte Fluoreszenzlicht durch den transparenten Trägerkörper hindurch an der dem planaren Wellenleiter und der Flußzelle gegenüberliegenden Seite des Reaktionsträgers bzw. Biochips durch eine, vorzugsweise einen Filter enthaltende Abbildungsoptik erfaßt und einem zugehörigen ortsauflösenden Detektor, z.B. einem Foto-Multiplier oder einer CCD-Kamera zum Auslesen des Reaktionsträgers, insbesondere Biochips, zugeführt wird.

Hierbei werden sämtliche Meßpunkte auf der Oberseite des planaren Wellenleiters des Reaktionsträgers bzw. Biochips gleichzeitig durch das evaneszente Feld des in den planaren Lichtwellenleiter eingekoppelten Anregungslichtes zur Fluoreszenzlichtemission in Verbindung mit einer Reaktion zwischen den immobilisierten Agenzien auf der Chip-Oberfläche, wie z.B. DNA-Einzelsträngen und den nachzuweisenden Substanzen in der Probe, wie z.B. DNA-Einzelsträngen, angeregt.

Weitere bevorzugte Ausführungsbeispiele des erfindungsgemäßen Verfahrens sind in den übrigen Unteransprüchen dargelegt.

Die vorgenannte Aufgabe wird ferner hinsichtlich der Einrichtung erfindungsgemäß gelöst durch eine Einrichtung zur Bestimmung temperaturabhängiger Parameter, wie der Assoziations-/Dissoziationsparameter und/oder der Gleichgewichtskonstante von aus mindestens zwei Komponenten gebildeten Komplexen mit einem Reaktionsträger, dessen optisch anregbare Oberfläche spezifisch an die ersten Komponenten bindende zweite Komponenten aufweist, die Meßpunkte auf dem Reaktionsträgers bilden, mit einer Einrichtung zum Inkontaktbringen der in der in der Flüssigphase befindlichen ersten Komponenten und der an den Reaktionsträger gekoppelten, spezifisch an die ersten Komponenten bindenden zweiten Komponenten, mit einer Einrichtung zum Temperieren der Meßpunkte, mit einer Lichtquelle zur Einkopplung von Anregungslicht zur Anregung der Emission von Fluoreszenzlicht in Abhängigkeit von der Bindung der ersten Komponenten an die zweiten Komponenten des Reaktionsträgers und mit einem Detektor zum Erfassen des emittierten Fluoreszenzlichtes zur Bestimmung der Bindung der ersten Komponenten an die zweiten Komponenten als Funktion der Temperatur.

Eine derartige Einrichtung kann neben der exakten Bestimmung der obengenannten Schmelzpunkte auch noch die Dissoziations-Assoziationskinetik von Nukleinsäuren über einen Temperaturgradienten messen, und kann durch die errechenbaren Gleichgewichtskonstanten nun eine klare Auskunft über die Reaktionsenthalpie geben und somit eine Konzentrationsbestimmung der Analysate ermöglichen. Selbst komplexere Hybridisierungskurven, z.B. mehrere Hybridisierungspartner an einer Sonde, das ist eine an die Festphase gebundene Nukleinsäure, sind durch mathematische Analyse der Hybridisierungskurve auswertbar, so daß Parameter, die bisher nicht chiptauglich waren, mit dieser Analysemethode nun erfaßt werden können.

Neben der Auslesung biochemischer Reaktionen ist die erfindungsgemäße Einrichtung auch für den Nachweis anorganischer Stoffe geeignet. Ein Beispiel hierfür ist die Gassensorik.

In der Vergangenheit wurden verschiedene Gassensoren vorgeschlagen, die sogenannte sensitive Beschichtungen verwenden. Dieses können z.B. Polymerfilme oder Sol-Gel-Schichten sein, die bestimmte Gase adsorbieren. Baut man in diese Schicht spezifisch mit dem Analyten reagierende Substanzen und/oder Indikatoren ein, ist eine Änderung einer Schichteigenschaft in Gegenwart bestimmter Gase detektierbar. Die betreffenden Schichteigenschaften können z.B. ein Farbumschlag, eine Dichte- oder Brechzahländerung oder eine Änderung der dielektrischen Eigenschaften betreffen.

Die erfindungsgemäße Einrichtung kann in ähnlicher Weise für die Gasdetektion eingesetzt werden, wenn entweder der Analyt fluoresziert oder eine Fluoreszenz der Beschichtung durch Aufnahme des Analyten unterdrückt wird ('Fluoreszenz-Quenching'). Die erfindungsgemäße optische Anordnung erlaubt in diesen Fällen den Nachweis einer großen Zahl von Analyten bei Verwendung mehrerer verschiedener Beschichtungen, die sich strukturiert auf der Oberfläche des Wellenleiters oder des Prismas befinden. Außerdem wird der zeitliche Verlauf der Reaktion nachgewiesen.

Eine wichtige Zusatzinformation erhält man aus der Temperatur-Abhängigkeit der Gasaufnahme der Beschichtung, da die nachzuweisenden Gase in der Regel bei erhöhter Temperatur wieder desorbieren. Die Kenntnis der Temperatur, bei der sich nur noch ein bestimmter, festzulegender Anteil der Gaskonzentration in dem Film befindet, erhöht die Spezifität des Sensors. Diese Information kann aber auch Aufschluss über den Zustand der Schicht (z.B. eine Alterung der Schicht) geben. Die Messdaten werden damit zuverlässiger. Insbesondere können die Sensordaten unabhängig von absoluten Fluoreszenzintensitäten erfasst werden. Nicht zuletzt besteht im Fall einer kontinuierlichen oder quasi-kontinuierlichen Messung in der Temperaturerhöhung die Möglichkeit, auch solche Gase aus der Schicht herauszutreiben, die bei einer Abnahme der Umgebunskonzentration nicht von selbst desorbieren. Die gleichzeitige Fluoreszenzmessung gibt Auskunft darüber, ob die Desorption des Gases erfolgt ist.

Das Wesen der Erfindung besteht in der Bestimmung temperaturabhängiger Parameter, wie der Assoziations-/Dissoziationsparameter und/oder der Gleichgewichtskonstante von aus mindestens zwei Komponenten gebildeten Komplexen, wobei die in einer Flüssigphase befindlichen ersten Komponenten mit Meßpunkten auf einem optisch anregbaren Reaktionsträger, gebildet durch an den festen Reaktionsträger gekoppelte, spezifisch an die ersten Komponenten bindende zweite Komponenten, in Kontakt gebracht werden und die Bildung oder der Zerfall der Komplexe als Funktion der Temperatur beobachtet wird.

Weitere, bevorzugte Ausgestaltungen der erfindungsgemäßen Einrichtung sind in den übrigen Unteransprüchen dargelegt.

Die Erfindung wird nachstehend anhand von Ausführungsbeispielen und zugehörigen Zeichnungen näher erläutert. In diesen zeigen:
- Fig. 1a: einen Biochip in schematisch perspektivischer Darstellung;
- Fig. 1b: einen Ausschnitt eines Biochips nach Fig. 1a für einen Meßpunkt einer Oberfläche mit immobilisierten DNA-Einzelsträngen;
- Fig. 1c: eine schematische Darstellung der Zugabe der Probe mit den zu analysierenden DNA-Einzelsträngen zu dem Meßpunkt nach Fig. 1a, und eine Darstellung der komplementären Interaktion zwischen den immobilisierten DNA-Einzelsträngen nach Fig. 1b und den in der Probe enthaltenen DNA-Einzelsträngen (Hybridisierung);
- Fig. 2: eine Darstellung der Trennung von gebundener und flüssiger Phase nach dem ATR-Prinzip;
- Fig. 3: eine schematische Darstellung der Einrichtung zum Auslesen eines ATR-Prismas durch Einfachreflexion;
- Fig. 4: eine schematische Darstellung zum Auslesen eines ATR-Prismas durch Vielfachreflexion;
- Fig. 5: eine schematische Darstellung der Einrichtung unter Anwendung des Prinzips einer "homogenisierten" Vielfachreflexion (Flächenausleuchtung);
- Fig. 6: eine schematische Darstellung der Einrichtung unter Verwendung eines planaren Lichtwellenleiters;
- Fig. 7: Graphen zur Darstellung der Fluoreszenzverteilung sowie deren Ableitung nach der Zeit; und
- Fig. 8: ein schematischer Aufbau einer Flusszelle mit Temperierung.

Als erstes Ausführungsbeispiel des Verfahrens und der Einrichtung, die den Gegenstand der vorliegenden Patentanmeldung bilden, wird die Gestaltung und das Auslesen eines Biochips gewählt, wie er für die Analyse von DNA-Sequenzen, die sich in einer Probe befinden, und zur Analyse der Nukleinsäure mit einer Oberfläche eines Biochips in Kontakt gebracht werden, verwendet.

Selbstverständlich ist dies nur ein Ausführungsbeispiel, und es können hier auch andere molekularbiologische, biologische und/oder chemische Substanzen, wie z.B. Gene und Antikörper, detektiert werden.

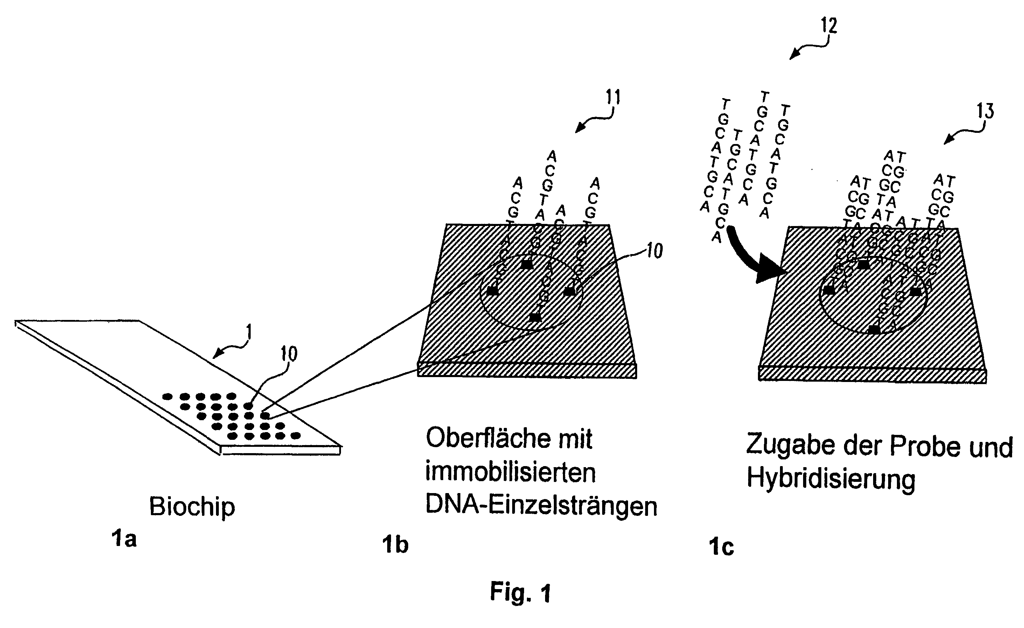

In Fig. 1a ist in schematischer Darstellung ein solcher Biochip 1 gezeigt, der ein kleines Plättchen bildet, auf dessen Oberfläche eine Vielzahl von Nukleinsäuren 11 in einzelnen Meßpunkten 10 immobilisiert sind. An jedem einzelnen Meßpunkt 10 befindet sich ein Oligonukleotid mit einer definierten Basensequenz. Dies ist in Fig. 1b dargestellt. In Fig. 1c sind die zu analysierenden Nukleinsäuren der zu untersuchenden Probe mit 12 bezeichnet und durch einen Pfeil ist angedeutet, daß diese in Kontakt gebracht werden mit den komplementären Nukleinsäuren 11, die sich im Meßpunkt 10 befinden. Da jeder Einzelstrang eines Nukleinsäuremoleküls 11 mit seinem komplementären Strang 12 (s. Fig. 1b) eine Bindung eingeht (Hybridisierung), erhält man nach Prüfung der einzelnen Meßpunkte 10 hinsichtlich der Anbindung von Probenmolekülen 12 eine Aussage über die in der Probe vorhandenen DNA-Sequenzen 12. Die hybridisierten DNA-Einzelstränge sind in Fig. 1c mit 13 bezeichnet.

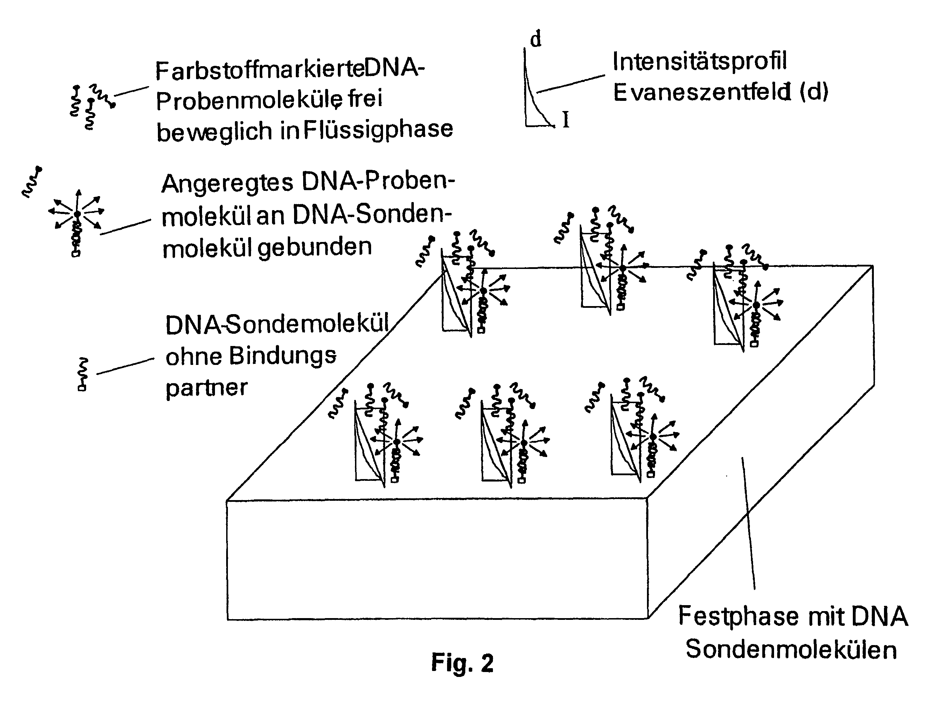

Die folgenden Ausführungsbeispiele verwenden entweder die abgeschwächte Totalreflexion (ATR) oder die Totale Interne Reflexions Fluoreszenz (TIRF). Durch die Totalreflexion eines Lichtstrahles an der Grenzfläche zwischen zwei optisch unterschiedlich dichten Medien wird im optisch dünneren Medium ein elektromagnetisches Feld erzeugt. Das optisch dichtere Medium sei hier eine Festphase und das optisch dünnere Medium eine Flüssigphase. Dieses sogenannte Evaneszentfeld dringt nur einige hundert nm von der Grenzfläche in das flüssige Umgebungsmedium ein. Dadurch werden fast ausschließlich die an der Oberfläche angebundenen Fluoreszenzfarbstoffe nachgewiesen. Die im Umgebungsmedium gelösten Farbstoffe tragen nur geringfügig zum Meßsignal bei, wie es in Fig. 2 dargestellt ist. Das ermöglicht die Messung des zeitlichen Verlaufs von Reaktionen.

In Fig. 2 ist deutlich dargestellt, daß die Intensitätsprofile der Evaneszentfelder sehr stark abfallen.

Dabei bringt eine beheizbare Durchflußzelle die Flüssigphase mit der Festphase in Kontakt und ermöglicht die Temperierung der Reaktionspartner. Eine Flußzelle 6 ist gekoppelt mit einem Fluidik-System zur Handhabung der Flüssigphase. Durch den permantenten Kontakt der Sonde, also der an die Festphase gebundenen Nukleinsäure, mit der flüssigen Phase, ist die Regenerierbarkeit des Biochips gegeben. Als Meßchip wird ein transparentes Prisma 5 verwandt.

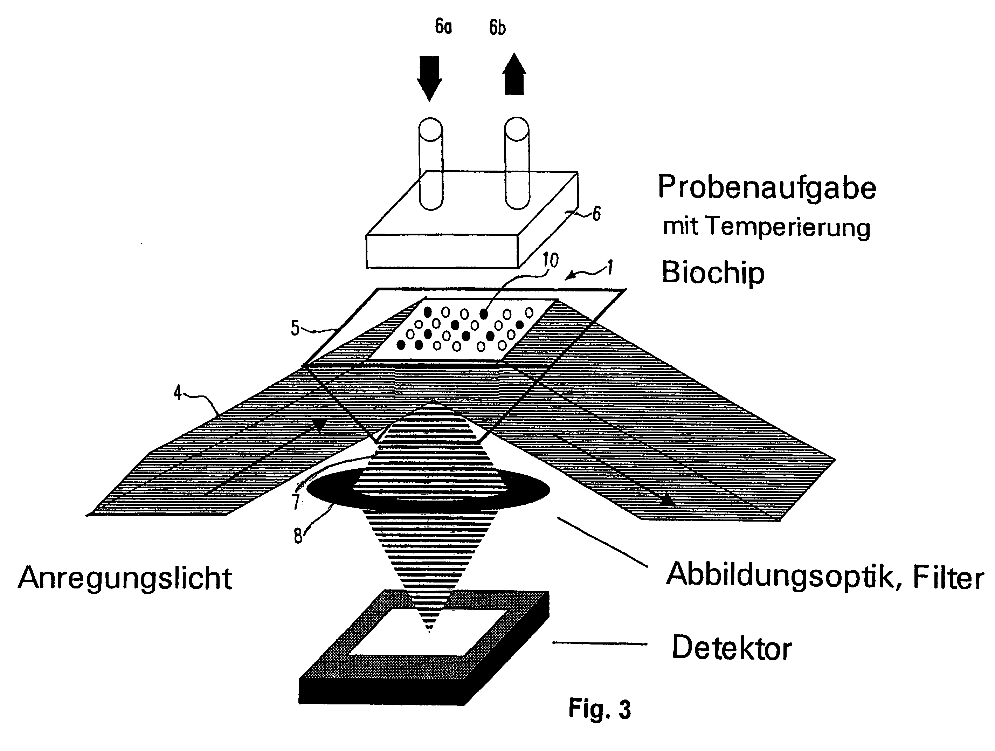

Bei der Anordnung nach Fig. 3 wird die Kante des Prismas 5 flächig ausgeleuchtet. Eine einfache Totalreflexion an der Grundseite des Prismas genügt, um eine ausreichend große Meßfläche zu erhalten.

Fig. 3 zeigt des weiteren in schematischer Darstellung eine Einrichtung zum Auslesen des Biochips 1, der eine Konfiguration aufweist, wie er bereits oben beschrieben worden ist. Der Biochip 1 besteht wiederum aus einem transparenten Substrat und vorzugsweise einer auf das Substrat aufgebrachten hochbrechenden Beschichtung als planarer Lichtwellenleiter. Der Lichtwellenleiter trägt ein Feld von Meßpunkten 10, und ein Anregungslicht 4 wird über das Prisma 5 in den Lichtwellenleiter eingekoppelt und in diesem geführt. Die Meßsonde ist so ausgebildet, wie dies anhand der Fig. 1b erläutert wurde.

Zur Analyse von den DNA-Nukleinsäuren in einer Probe wird diese Probe hier in der Flußzelle 6 und durch diese hindurchgeführt, wie dies durch die Strömungspfeile 6a und 6b angedeutet ist. Die Flußzelle 6 wird abgedichtet auf den Lichtwellenleiter aufgesetzt und umgibt das Meßfeld mit den Meßpunkten 10 rahmenartig und fluiddicht, so daß die Probe mit allen Meßpunkten 10 zur möglichen Hybridisierung in Wechselwirkung treten kann. Die Detektion der durch das Evaneszentfeld des Anregungslichtes 4 angeregten Fluoreszenzstrahlung 7 erfolgt z.B. mittels einer Abbildungsoptik 8 in Verbindung mit einem hier nicht gezeigten Filter und einem ortsauflösenden Detektor 9, wie z.B. einer CCD-Kamera oder einem Foto-Multiplier.

Auf diese Weise ist eine Erfassung der Hybridisierung und eine parallele Auslesung des Biochips 1 mit allen Meßpunkten 10 gleichzeitig möglich. Zugleich erfolgt eine selektive Anregung der gebundenen Fluorochrome in den Meßpunkten 10. Durch dieses Meßprinzip ist daher ohne besondere Probenvorbereitung eine mit großer Genauigkeit hinsichtlich der Ortsauflösung und auch hinsichtlich der Präsenz von hybridisierten Nukleinsäuren eine sehr schnelle Auswertung und Detektion des Biochips 1 möglich.

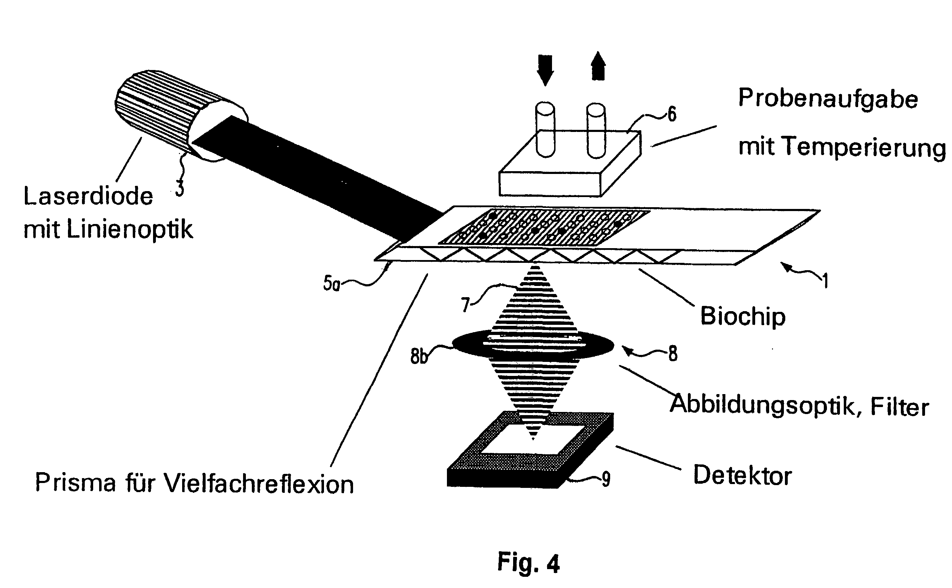

Bei der Anordnung gemäß Fig. 4 wird eine Kante eines wesentlich dünneren Prismas 5a, mit einer Stärke von ungefähr 1 mm mit einer Linienoptik ausgeleuchtet. Dabei sind mehrere Prismen 5a nebeneinander angeordnet. Durch Vielfachreflexion an den Ober- und Unterseiten der Prismen 5a wird eine flächige Ausleuchtung des Meßfeldes erzeugt. Die Detektion der emittierten Fluoreszenzstrahlung erfolgt mit einem ortsauflösenden Detektor 9. In diesem Fall dient als Lichtquelle eine Laserdiode 3.

Die Onlinemessung der Hybridisierung mittels eines ATR-Analysators mit beheizbarer Fluidik geschieht wie folgt.

Zunächst wird die Schmelzpunktbestimmung einer DNA beschrieben. Dabei können Sonden mit synthetischen, d.h. Oligonukleotide, oder natürlichen Proben, d.h. cDNAs, hybridisiert werden. Die Aufnahme der Signalintensität bei verschiedenen Temperaturen erlaubt es, den Schmelzpunkt Tm der DNA zu bestimmen. Dies ist die Temperatur, bei der 50 % der maximalen Signalstärke erreicht werden. Dieser Versuch kann mit einer Vielzahl von Sonden durchgeführt werden.

Als nächstes wird die Bestimmung der Bildungskonstante beschrieben. Bei einer bekannten Konzentration von Analysatmolekülen kann nach Massen-Wirkungs-Gesetz die Geschwindigkeitskonstante einer Reaktion gemessen werden. Dies kann auf dem Chip mit einer Vielzahl von Analysepunkten geschehen.

Sind der Schmelzpunkt und die Bildungskonstante bekannt, so kann die Konzentration eines oder mehrerer Analyte in einer komplexen Probe über die Aufnahme der Hybridisierungskurve bestimmt werden.

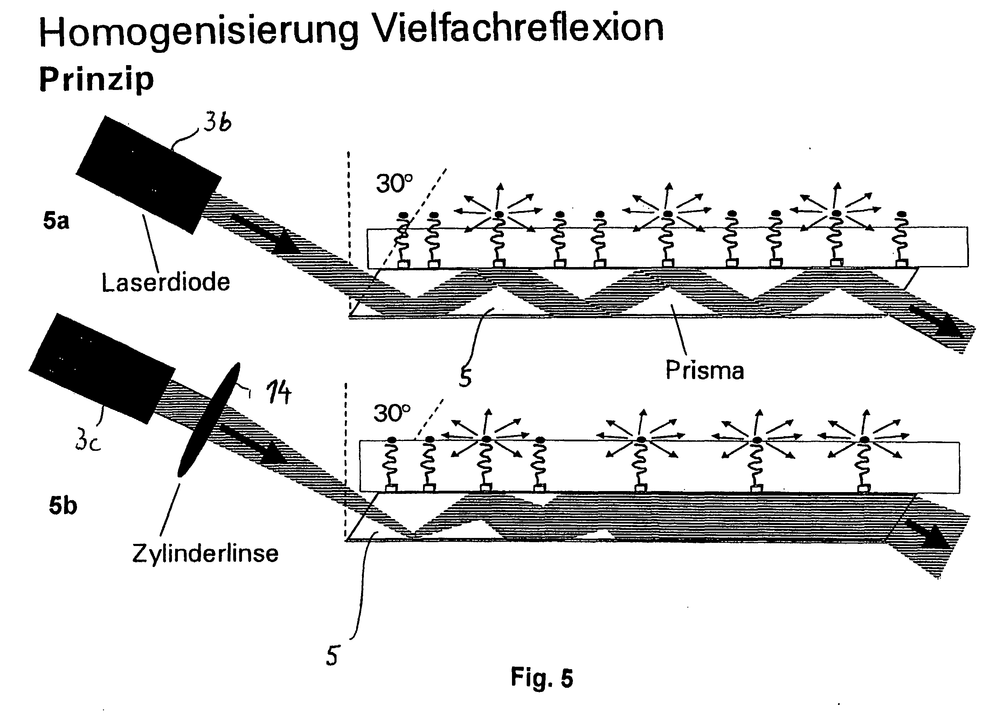

Fig. 5 zeigt in den Fig. 5a und 5b jeweils die Einstrahlungsverhältnisse in ein Prisma 5 als Reaktionsträger unter Darstellung des Laserstrahles mit Vielfachreflexion im ATR-Primsa 5 (Fig. 5a) unter Einstrahlung durch eine Laserdiode 3a und in Fig. 5b die Möglichkeit, tatsächlich zu einer flächigen Ausleuchtung der Oberfläche des Prismas 5 dadurch zu gelangen, daß die Einstrahlung des Lichtes durch die Lichtquelle 3c über eine Zylinderlinse 14 in das Prisma 5 erfolgt.

Wie dargestellt, wird hierdurch eine tatsächlich im wesentlichen vollflächige Ausleuchtung der Prismenoberfläche erreicht, während bei der Ausleuchtung des Randes des Prismas 5 mit einem kollimierten Laserstrahl dieses dazu führt, daß die Ober- und Unterseite des Prismas 5 nur an bestimmten Stellen selektiv ausgeleuchtet wird und Meßpunkte, die in nicht ausgeleuchteten Bereichen liegen, nicht ausgewertet werden können. Demgegenüber wird bei der Fokussierung des Lichtstrahles mit einer Zylinderlinse 14 auf die Kante des Prismas 5 erreicht, daß der Lichtstrahl im Prisma 5 divergent weiterläuft. Nach einer gewissen Wegstrecke ist der Strahl so weit aufgeweitet, daß praktisch eine homogene Ausleuchtung der Biochip-Oberfläche gegeben ist.

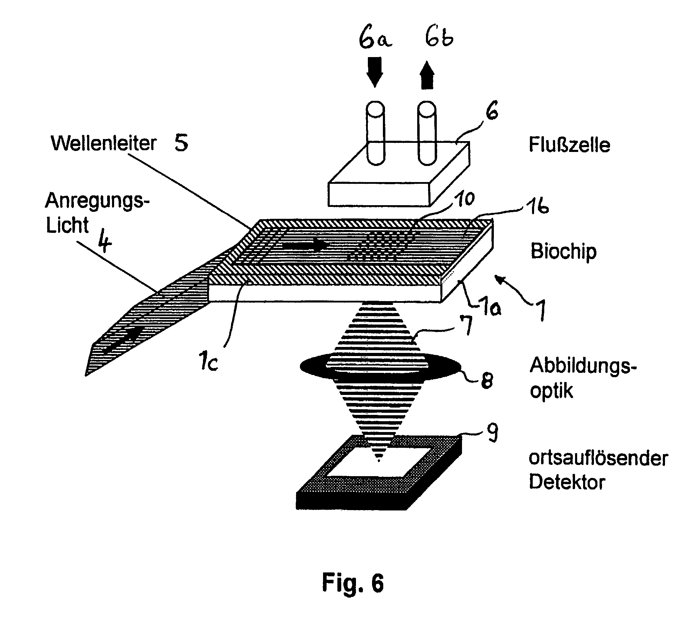

Fig. 6 zeigt in schematischer Darstellung eine Einrichtung zum Auslesen eines Biochips 1, der eine Konfiguration aufweist, wie sie in Fig. 2 dargestellt ist. Der Biochip 1 besteht wiederum aus dem transparenten Substrat 1a und der auf das Substrat aufgebrachten hochbrechenden Beschichtung als planarer Lichtwellenleiter 1b. Die Ränder 1c des Biochips 1 bleiben außerhalb der Wellenleiterstruktur. Der Lichtwellenleiter trägt ein Feld von Meßpunkten 10 und das Anregungslicht 4 wird über ein Koppelgitter 5 in den Lichtwellenleiter 1b eingekoppelt und in diesem geführt. Die Meßpunkte 10 sind so ausgebildet, wie dies anhand der Fig. 1b und Fig. 2 erläutert wurde. Zur Analyse von DNA-Nukleinsäuren in einer Probe wird diese Probe hier beispielsweise in einer Flußzelle 6 und durch diese hindurchgeführt, wie dies durch die Strömungspfeile 6a, 6b angedeutet ist. Die Flußzelle 6 wird abgedichtet auf den Lichtwellenleiter 1b aufgesetzt und umgibt das Meßfeld mit den Meßpunkten 10 rahmenartig und fluiddicht, so daß die Probe mit allen Meßpunkten 10 zur möglichen Hybridisierung in Wechselwirkung treten kann. Die Detektion der durch das Evaneszentfeld des Anregungslichtes angeregten Fluoreszenzstrahlung 7 erfolgt z.B. mittels einer Abbildungsoptik 8 in Verbindung mit einem (hier nicht gezeigten) Filter und einem ortsauflösenden Detektor 9, wie z.B. einer CCD-Kamera oder einem Foto-Multiplier. Die Detektion kann aber auch durch Abstrahlung des Fluoreszenzlichtes nach oben oberhalb des Wellenleiters 1b erfolgen.

Auf diese Weise ist eine "in-situ-Erfassung" der Hybridisierung und eine parallele Auslesung des Biochips 1 mit allen Meßpunkten 10 gleichzeitig möglich. Zugleich erfolgt eine selektive Anregung der gebundenen Fluorochrome in den Meßpunkten. Durch dieses Meßprinzip ist daher ohne besondere Probenvorbereitung eine mit großer Genauigkeit hinsichtlich der Ortsauflösung und auch hinsichtlich der Präsenz von hybridisierten Nukleinsäuren eine sehr schnelle Auswertung und Detektion des Biochips 1 möglich.

Bekanntermaßen muß bei einer Analysenanordnung nach Fig. 6 zusätzlicher Aufwand für die Einkopplung des Anregungslichtes 4 in den Wellenleiter 1b (hier über ein Gitter 5) getrieben werden. Einerseits werden somit zusätzliche Präparationsschritte bei der Herstellung des Biochips 1, andererseits sind Justiervorrichtungen in der optischen Anordnung zwischen Anregungslichtquelle (Laserquelle) und Biochip erforderlich. Die damit einhergehende Erhöhung der Biochipkosten, die als Verbrauchsmaterial problematisch ist, kann dadurch vermieden werden, daß eine Meß- und Analysenanordnung nach der schematischen Darstellung gemäß Fig. 4 verwendet wird, bei der eine Anregung der Fluoreszenz auf der Oberseite des Lichtwellenleiters 1b z.B. von der Rückseite des Biochips 1 und damit von der gegenüberliegenden Seite in Bezug auf die Flußzelle 6 erfolgt, aber eine Detektion des von den gebundenen Fluorochromen emittierten Fluoreszenzlichtes durch Einkopplung desselben in den planaren Wellenleiter 1b vorgesehen wird.

Als alternative Lösung wird das von einer Laserstrahlquelle emittierte Anregungslicht vorzugsweise über eine Ablenkeinheit 14 auf einen Umlenkspiegel 15 und von dort in den Wellenleiter 1b im Bereich des Meßfeldes der Meßpunkte 10 geführt, an dem sich z.B. die Flußzelle 6 befindet. Hierbei findet mit einer Scanneinrichtung eine zweiachsige Relativbewegung zwischen Anregungslichtstrahl und Biochip statt. Auch in diesem Fall wird durch den planaren Wellenleiter 1b eine Trennung von angebundenen und gelösten Fluorochromen erreicht, da nur das im Bereich des Evaneszentfeldes des Anregungslichtes 4 emittierte Fluoreszenzlicht 7 auch in den planaren Wellenleiter 1b eingekoppelt und anschließend detektiert wird. Die gelösten Fluorochrome erzeugen keine Untergrundintensität, da dieses Licht nicht an die Erfassungseinrichtung herangeführt wird, sondern nur das in dem planaren Wellenleiter 1b geführte Fluoreszenzlicht der gebundenen Fluorochrome. Die Strömungspfeile 6a, 6b geben wiederum den Probenfluß durch die Flußzelle 6 an, während die Abbildungsoptik 8 mit Filter im Anschluß an den planaren Lichtwellenleiter 1b dargestellt ist, wobei als ortsauflösender Detektor hier ein Foto-Multiplier verwendet wird.

Da bei diesem Ausführungsbeispiel eine zeilenweise Auslesung erfolgen muß, wird der Biochip 1 in Pfeilrichtung A, wie angegeben, entsprechend bewegt.

Gegebenenfalls kann auch auf der anderen Seite des Wellenleiters eine Erfassungseinrichtung mit Auswerteoptik, Filter und Foto-Multiplier zur Erfassung des auf der anderen Seite aus dem Lichtwellenleiter 1b austretenden Fluoreszenzlichtes vorgesehen sein oder der Detektor kann direkt an die Kante des Lichtwellenleiters 1b angekoppelt sein.

Als Lichtwellenleiter kann auch unmittelbar eine Glasplatte verwendet werden, die selbst den Reaktionsträger und zugleich planaren Lichtwellenleiter bildet. In diesem Fall kann eine separate, den Lichtwellenleiter bildende Beschichtung eines Substrats entfallen.

Die erfindungsgemäße Lösung gestattet die Real-Time-Messung und Auswertung von Biochips oder auch von anderen Reaktionsträgem, auf deren mit einem planaren Wellenleiter beschichteten Oberseite durch Reaktion Analysen von Substanzen in Proben bewirkt werden.

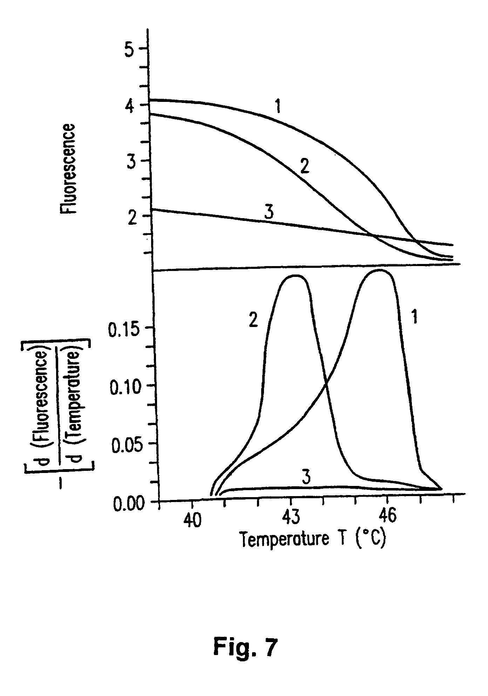

Unter Bezugnahme auf die Fig. 7 wird nunmehr ein weiteres Ausführungsbeispiel beschrieben.

Durch Veränderung der Temperatur können die Schmelzpunkte von immobilisierten Oligonukleotiden ermittelt werden, da man ein Ablösen bzw. Binden der Probe bei Erhöhen bzw. Verringern der Temperatur beobachten und aus der daraus erstellbaren Schmelzkurve rechnerisch den Schmelzpunkt ermitteln kann. Die Schmelzpunktbestimmung kann in einem auf dem Chip parallelisierten Ansatz für viele Oligonukleotide gleichzeitig erfolgen.

Zur Schmelzkurvenbestimmung wurden z.B. Oligonukleotide eingesetzt, deren Sequenz einem Abschnitt des Hämochromatosegens entsprechen. Dabei wurden sowohl Oligonukleotide verschiedener Längen als auch verschiedene Basensubstitutionen an unterschiedlichen Stellen getestet. Die synthetisch hergestellten Oligonukleotide waren dabei am 5'-Ende je mit einem Spacer aus 10 Thymin-Basen und einem C6-Amino-linker versehen. Über die Aminogruppe am Linker wurden die Oligonukleotide an silanisierte Glasobjektträger kovalent gekoppelt. Zur Detektion wurde entweder mit komplementären fluoreszenzmarkiertem (Cy5) Oligonukleotid oder PCR-Produkt aus Hämochromatosepatienten hybridisiert und im ATR-Reader bei Temperaturerhöhung die Dissoziationskinetik gemessen. Dabei ergab sich z.B. für Oligonukleotide einer Länge von 17 Basen, die in einer Konzentration von 2µM auf einem Glas-Chip immobilisiert worden waren und an zentraler Position entweder die dem Wildtyp entsprechende Base G (5' ATATACGTGCCAGGTGG 3'; SEQ ID NO:1), die der Kurve 1 von Fig. 5 entspricht, bzw. die der Mutanten entsprechende Base A (5' ATATACGTACCAGGTGG 3'; SEQ ID NO:2), die der Kurve 2 von Fig. 5 entspricht, enthielten, bei Hybridisierung mit einem komplementären äquimolaren Oligonukleotid der Länge von 31 Basen bei Raumtemperatur und nachfolgender Temperaturerhöhung folgende Schmelzpunkte: Von den Oligonukleotiden, die eine Missense-Base enthielten, dissoziierte das komplementäre Oligonukleotid früher ab (Tm 43°C) als von dem dem Wildtyp entsprechenden Oligonukleotid (Tm 46°C).

Die Fluoreszenz nimmt durch das Ablösen des fluoreszenzmarkierenden komplementären Oligonukleotids von der Oligonukleotidsonde bei Temperaturerhöhung ab.

Dies ist in dem oberen Graph von Fig. 5 für den immobilisierten Wildtyp-Oligonukleotids mit der Kurve 1 dargestellt, während die Kurve 2 dem immobilisierten Oligonukleotid, das die Missense-Base enthält und den Mutanten entspricht, gezeigt.

Die Kurve 3 dient der Kontrollmessung und zeigt die Hybridisierung ohne Oligonukleotid.

Der untere Graph der Fig. 5 zeigt die Ableitung der Fluoreszenz nach der Zeit. Dabei ist aus Übersichtlichkeitsgründen die Ableitung nach Vorzeichenumkehr aufgetragen.

Nachstehend wird ein Ausführungsbeispiel zur Bestimmung der Temperaturabhängigkeit der Gleichgewichtskonstante von Protein/Protein- bzw. Protein/Ligand-Komplexen beschrieben.

Das Prisma wurde mit einer Aminosilan-Gruppe gemäß bekannter Verfahren beidseitig silanisiert. Die zu koppelnden Proteine und Liganden wurden unter Verwendung des Carbodiimid-NHS Verfahrens aktiviert, das zu aktivierten Carboxy-Gruppen der Proteine und Liganden führt.
Die aktivierten Proteine und Liganden wurden auf das Prisma in Array-Anordnung mit einem Nadeldrucker (Pinprinter) aufgetragen. Nach dem Auftragen wurde das Prisma in einer feuchten Kammer bei 37°C zwei Stunden inkubiert. Das Prisma wurde dann 30 min bei Raumtemperatur in Borat-Puffer pH 9,5 zur Hydrolyse der verbliebenen aktiven Ester-Gruppen und danach eine Stunde bei Raumtemperatur in 1 % BSA (w/v) in 100 mM PBS pH 7.4 zur Blockierung der Prisma-Oberfläche gegenüber unspezifischer Bindung inkubiert.
Der Analyt (Proteine und Liganden) wurde mit dem Cy5-Markierungs-Kit (Pharmacia) gemäß Hersteller-Anleitung Fluoreszenz-markiert.
Das Prisma wurde danach in das ATR-Detektorelement eingebaut und die Flusszelle mit PBS gespült und sodann mit 1 mM Fluoreszenz-markiertem Analyten gefüllt. Nach Erreichen des Gleichgewichtszustandes wurde eine 30 s Aufnahme durchgeführt.
Die Temperatur der Flusszelle wurde stufenweise mit X°C pro Minute erhöht. 30 s Aufnahmen wurden jeweils nach X min durchgeführt. Nach Erreichen der gewünschten Temperatur wurden die einzelnen Meßpunkte in dem Array mit der SignalseDemo Software (GeneScan) quantifiziert. Zur Bestimmung der Temperatur-Abhängigkeit der Gleichgewichtskonstante wurde in die Messdaten mit Hilfe des Programmes Grafit (Erithacus Software) eine geeignete Regressionsfunktion gelegt.

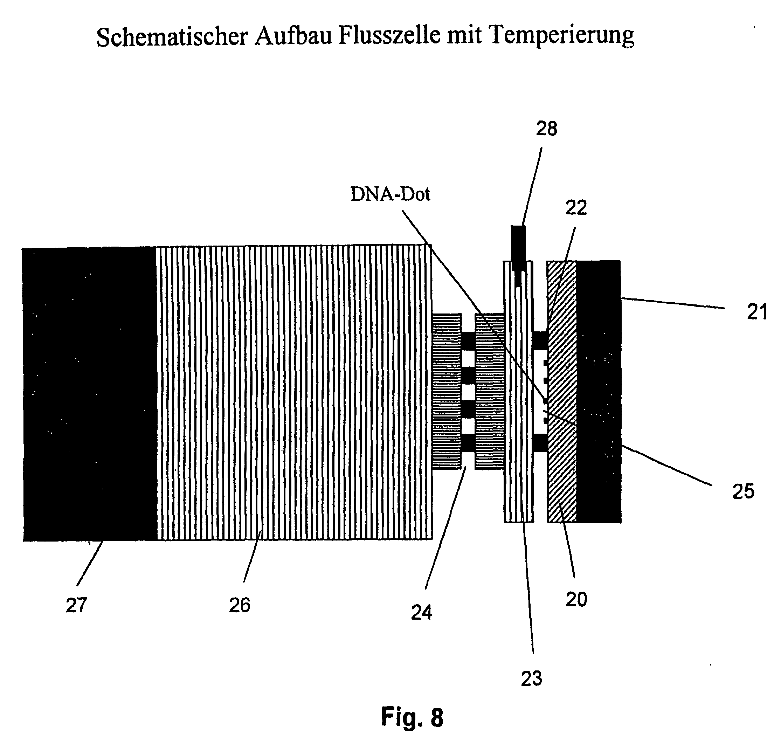

Die Figur 8 zeigt die Temperierung der Flußzelle.

Für die Aufnahme von DNA-Schmelzkurven ist eine homogene Temperierung der Sonden-Proben-Hybride in der Flußzelle erforderlich. Ein großer Temperaturbereich muß von der Temperiereinheit abgedeckt werden können, um den Schmelzpunkt von sowohl sehr kurzen als auch sehr langen Nukleotidsträngen messen zu können. Der Temperaturbereich zwischen 0°C und 100°C ist mit einer Peltiertemperierung leicht realisierbar. Die Verwendung eines Peltierelementes (24) ermöglicht außerdem einen sehr kompakten Aufbau. Abbildung 8 zeigt den schematischen Aufbau der temperierbaren Flußzelle. Der Biochip (20) wird mit einem Chiphalter (21) an die Flußzelle gepresst. Eine Vertiefung in der Flußzelle bildet das Reaktionsvolumen (25), welches durch eine O-Ring Dichtung (22) abgedichtet wird. Die Rückseite (23) der Flußzelle wird zur Temperierung mit einem Peltierelement (24) in Kontakt gebracht. Der Wärmeaustausch mit der Umgebung wird durch einen Kupferblock (26) mit Lüfter (27) realisiert. Ein Widerstandsthermometer (28), das in der Flußzelle eingebaut ist, wird zur Temperaturmessung verwendet und bildet zusammen mit einem PID-Regler und dem Peltierelement (24) einen Regelkreis.

## Patentansprüche

1. Verfahren zur parallelen Bestimmung temperaturabhängiger Parameter, wie der Assoziations-/Dissoziationsparameter und/oder der Gleichgewichtskonstante von aus mindestens zwei Komponenten gebildeten Komplexen, wobei
die in einer Flüssigphase befindlichen ersten Komponenten (12) mit einer Vielzahl von Messpunkten (10) auf einem optisch anregbaren Reaktionsträger gebildet durch an den festen Reaktionsträger gekoppelte, spezifisch an die ersten Komponenten (12) bindende zweite Komponenten (11), in Kontakt gebracht werden durch Inkontakt-Bringen der Flüssigphase und des Reaktionsträgers (1) unter Bildung von Komplexen (13) und
die Anregung von an die ersten Komponenten (12) und/oder zweiten Komponenten (11) gebundenen, fluoreszierenden oberflächennahen Farbstoffen zur Emission von Fluoreszenzlicht (7) durch eingestrahltes Anregungslicht (4) und Detektion des emittierten Fluoreszenzlichtes in einem variablen Temperaturfeld erfolgt und
die Bildung oder der Zerfall der Komplexe umfassend erste Komponenten (12) und zweite Komponenten (11) als Funktion der Temperatur beobachtet wird.

2. Verfahren nach einem der vorhergehenden Anspruche, **dadurch gekennzeichnet; dass** die ersten und/oder zweiten Komponenten Oligo- oder Polypeptide sind.

3. Verfahren nach einem der vorhergehenden Anspruche, **dadurch gekennzeichnet, dass** die ersten und/oder zweiten Komponenten Nukleinsäure-Einzelstrange sind.

4. Verfahren nach einem der vorhergehenden Ansprüche, **dadurch gekennzeichnet, dass** das Anregungslicht (4) in einen, vorzugsweise Lichtwellenleiter mittels einer optischen Einrichtung, wie z.B. einem oder mehreren Prismen (5; 5a) eingekoppelt wird.

5. Verfahren nach einem der vorhergehenden Anspruche, **dadurch gekennzeichnet, dass** das Anregungslicht (4) durch Totalreflexion (ATR) oder Totale Interne Reflexions-Fluoreszenz (TIRF) der Lichtstrahlen an einer Grenzfläche zwischen zwei optisch unterschiedlich dichten Medien im optisch dünneren Medium ein elektromagnetisches Feld wird, wobei das optisch dichtere Medium eine Festphase und das optisch dünnere Medium eine Flüssigphase ist zur Messung des zeitlichen Verlaufs der Reaktion.

6. Verfahren nach Anspruch 4 oder 5, **dadurch gekennzeichnet, dass** Prismen (5a) vorgesehen sind, die durch Vielfachreflexion an der Ober- und Unterseite der Prismen (5a) mittels einer Linienoptik eine flächige Ausleuchtung eines Messfeldes erzeugen.

7. Verfahren nach zumindest einem der vorhergehenden Ansprüche 1 bis 6, **dadurch gekennzeichnet, dass** mit Hilfe von in den planaren Lichtwellenleiter eingekoppelten Anregungslichtes (4) eine gleichzeitige Anregung aller Messpunkte (10) erfolgt.

8. Verfahren nach Anspruch 5, **dadurch gekennzeichnet, dass** das Fluoreszenzlicht (7) der spezifisch an die ersten Komponenten (12) bindenden zweiten Komponenten (11) vorzugsweise mittels einer einen Filter (8a) enthaltenden Abbildungsoptik einem •ortsauflosenden Detektor (9) oberhalb Oder unterhalb des Biochips (oder auf der Seite zum Auslesen des Biochips (1) zugeführt wird.

9. Verfahren nach einem der vorhergehenden Ansprüche, **dadurch gekennzeichnet, dass** die Flüssigphase entgast wird.

10. Verfahren nach zumindest einem der Ansprüche 1 bis 9, **dadurch gekennzeichnet, dass** das Fluoreszenzlicht durch das evanezente Feld von in einen planaren Lichtwellenleiter eingekoppelten Anregungslicht, insbesondere Laserlicht, erzeugt wird.

11. Verfahren nach zumindest einem der vorhergehenden Ansprüche 1 bis 9, **dadurch gekennzeichnet, dass** das Fluoreszenzlicht durch Anregungslicht, insbesondere Laserlicht, aus der Umgebung eines die Messpunkte tragenden optischen Elementes ist erzeugt wird.

12. Einrichtung zur Bestimmung von temperaturabhängigen Parametern, wie der Assoziations-/Dissoziationsparameter und/oder der Gleichgewichtskonstante von aus mindestens zwei Komponenten gebildeten Komplexen mit einem Reaktionsträger (1), dessen optisch anregbare Oberfläche spezifisch an die ersten Komponenten (12) bindende zweite Komponenten (11) aufweist, die eine Vielzahl von Messpunkten (10) auf einem Reaktionsträger bilden, mit einer Einrichtung (6) zum Inkontaktbringen der in der Flüssigphase befindlichen ersten Komponenten (12) und der an den Reaktionsträger gekoppelten, spezifisch an die ersten Komponenten bindenden zweiten Komponenten (11), mit einer Einrichtung zum Temperieren der Messpunkte, mit einer Lichtquelle (3) zur Einkopplung von Anregungslicht (4) zur Anregung der Emission von Fluoreszenzlicht (7) in Abhängigkeit von der Bindung der ersten Komponenten (12) an die zweiten Komponenten (11) des Reaktionsträgers (1) und mit einem Detektor (9) zum Erfassen des emittierten Fluoreszenzlichtes (7) zur Bestimmung der Bindung der ersten Komponenten (12) an die zweiten Komponenten (11) als Funktion der Temperatur.

13. Einrichtung nach Anspruch 12, **gekennzeichnet durch** optische Einrichtungen zum Einkoppeln des Anregungslichtes (4) in einen, insbesondere planaren Lichtwellenleiter.

14. Einrichtung nach Anspruch 13, **dadurch gekennzeichnet, dass** die optischen Einrichtungen ein oder mehrere Prismen (5; 5a) sind.

15. Einrichtung nach Anspruch 14, **dadurch gekennzeichnet, dass** das Prisma oder die Prismen (5; 5a) dazu ausgelegt sind, das Licht einfach Oder vielfach zu reflektieren.

16. Einrichtung nach Anspruch 12 oder 15, **dadurch gekennzeichnet, dass** die Festphase aus Glas oder transparentem Kunststoff besteht.

17. Einrichtung nach zumindest einem der vorhergehenden Ansprüche 12 bis 16, **gekennzeichnet durch** eine in der Einrichtung integrierte Entgasungseinheit zur Entgasung der Flüssigphase.

18. Einrichtung nach zumindest einem der vorhergehenden Ansprüche 12 bis 15, **dadurch gekennzeichnet, dass** die Einrichtung zum Inkontaktbringen der ersten und zweiten Komponenten temperierbar, insbesondere beheizbar ist.

19. Einrichtung nach zumindest einem der vorhergehenden Anspruche 12 bis 16, **dadurch gekennzeichnet, dass** die Einrichtung (6) zum Inkontaktbringen der ersten Komponenten (12) mit den zweiten Komponenten (11) eine Flusszelle, eine Küvette oder ein Probenbehälter in abdichtender Verbindung im Bereich der Messpunkte auf der Oberflache des planaren Lichtwellenleiters (1b) des Reaktionsträgers (1) ist.

20. Einrichtung nach zumindest einem der vorhergehenden Anspruche 12 bis 19, **da-durch gekennzeichnet,** dass der Reaktionsträger ein Biochip (1) mit einem planaren Lichtwellenleiter auf seiner Oberseite ist, der die Messpunkte (10) trägt.

21. Einrichtung nach zumindest einem der vorhergehenden Anspruche 12 bis 20, **da-durch gekennzeichnet,** dass der Reaktionsträger (1) eine Glasplatte ist, die selbst den planaren Lichtwellenleiter bildet.

22. Einrichtung nach zumindest einem der vorhergehenden Anspruche 12 bis 21, **dadurch gekennzeichnet, dass** das Anregungslicht (4) von einer Seite des Reaktionsträgers her auf diesen fällt, und dass von den an der Oberflache des planaren Lichtwellenleiters gebundenen Fluorochromen emittierte Fluoreszenzlicht (7) im Bereich des evaneszenten Feldes des Anregungslichtes (4) in den planaren Lichtwellenleiter eingekoppelt und in diesem geführt und durch die Erfassungseinrichtung (8;9), die an zumindest einer Endfläche des planaren Lichtwellenleiters (1) angeordnet ist, erfassbar ist.

23. Einrichtung nach Anspruch 22, **dadurch gekennzeichnet, dass** die Erfassungseinrichtung eine Abbildungsoptik (8) mit einem Filter (8b) sowie einen Detektor (9) aufweist.

24. Einrichtung nach Anspruch 23, **dadurch gekennzeichnet, dass** der Detektor (9) ein Foto-multipler Oder eine CCD-Kamera ist.

25. Einrichtung nach zumindest einem der vorhergehenden Anspruche 12 bis 24, **dadurch gekennzeichnet, dass** eine Scanneinrichtung zum Auslesen des Reaktionsträgers (1) vorgesehen und der Reaktionsträger (1) und/oder das Anregungslicht aus der Umgebung des Reaktionsträgers (1) relativ zu diesem in zumindest einer Ebene bewegbar ist.

26. Einrichtung nach zumindest einem der Anspruche 12 bis 25, **dadurch gekennzeichnet, dass** die Einrichtung zum Inkontaktbringen der ersten und zweiten Komponenten beheizbar ist.

27. Einrichtung nach zumindest einem der vorhergehenden Anspruche 12 bis 26, **da-durch gekennzeichnet,** dass der Reaktionsträger einen, insbesondere planaren Lichtwellenleiter tragt, an dessen Oberflache sich die Messpunkte befinden.

28. Einrichtung nach einem der Anspruche 12 bis 27, **dadurch gekennzeichnet, dass** ein Biochip (20) gegen eine Flusszelle gepresst ist und ein Reaktionsvolumen (25), gebildet zwischen Flusszelle und Biochip, durch einen O-Ring abgedichtet ist.

29. Einrichtung nach Anspruch 28, **dadurch gekennzeichnet, dass** eine Temperiereinrichtung für das Reaktionsvolumen durch ein Peltierelement (24) gebildet ist.

30. Einrichtung nach Anspruch 29, **dadurch gekennzeichnet, dass** das Peltierelement mit einer Ruckseite der Flußzelle in Kontakt ist.

31. Einrichtung nach einem Anspruche 28 bis 30, **dadurch gekennzeichnet, dass** in Verbindung mit dem Peltierelement ein wärmeleitfähiger Metallkörper, insbesondere ein Kupferblock ist, dessen Wärmeaustausch mit der Umgebung vorzugsweise durch ein nachgeschaltetes Lüfterelement (27) beeinflusst ist.

32. Einrichtung nach einem der Anspruche 29 bis 31, **dadurch gekennzeichnet, dass** die Flusszelle einen Temperaturfühler, insbesondere ein Widerstandsthermometer aufweist, der in Verbindung mit einem Regler, insbesondere PID-Regler, und dem Peltierelement einen Regelkreis bildet.

## Claims

1. Method for the parallel determination of temperature-dependent parameters, such as the association/dissociation parameters and/or the equilibrium constant of complexes formed from at least two components, wherein
the first components (12) in a liquid phase are brought into contact with a large number of measurement points (10) on an optically excitable reaction carrier formed by second components (11), which are coupled to the solid reaction carrier and bond specifically to the first components (12), by bringing the liquid phase and the reaction carrier (1) into contact, with the formation of complexes (13), and
the excitation of fluorescent dyestuffs, which are bonded to the first components (12) and/or second components (11) and are close to the surface, to emission of fluorescent light (7) by incident exciting light (4) and detection of the fluorescent light emitted takes place in a variable temperature field and
the formation or the dissociation of the complexes comprising the first components (12) and the second components (11) is observed as a function of the temperature.

2. Method according to one of the preceding claims, **characterized in that** the first and/or second components are oligo- or polypeptides.

3. Method according to one of the preceding claims, **characterized in that** the first and/or second components are nucleic acid single strands.

4. Method according to one of the preceding claims, **characterized in that** the exciting light (4) is coupled into a preferably light wave conductor by means of an optical device, such as e.g. one or more prisms (5; 5a).

5. Method according to one of the preceding claims, **characterized in that** the exciting light (4), by total reflection (ATR) or total internal reflection fluorescence (TIRF) of the light rays at an interface between two media of different optical density, generates an electromagnetic field in the medium of lower optical density, the medium of higher optical density being a solid phase and the medium of lower optical density being a liquid phase, for measurement of the course of the reaction with respect to time.

6. Method according to claim 4 or 5, **characterized in that** prisms (5a) which generate a planar illumination of a measurement field by multiple reflection on the upper and under-side of the prisms (5a) by means of a line lens system are provided.

7. Method according to at least one of the preceding claims 1 to 6, **characterized in that** a simultaneous excitation of all the measurement points (10) takes place with the aid of exciting light (4) coupled into the planar light wave conductor.

8. Method according to claim 5, **characterized in that** the fluorescent light (7) of the second components (11) which bond specifically to the first components (12) is preferably led by means of an imaging lens system containing a filter (8a) to a position-resolving detector (9) above or below the biochip (or on the side for reading the biochip (1).

9. Method according to one of the preceding claims, **characterized in that** the liquid phase is degassed.

10. Method according to at least one of claims 1 to 9, **characterized in that** the fluorescent light is generated by the evanescent field of exciting light, in particular laser light, coupled into a planar light wave conductor.

11. Method according to at least one of the preceding claims 1 to 9 **characterized in that** the fluorescent light is generated by exciting light, in particular laser light, from the surroundings of an optical element which carries the measurement points.

12. Device for determination of temperature-dependent parameters, such as the association/dissociation parameters and/or the equilibrium constant of complexes formed from at least two components, with a reaction carrier (1), the optically excitable surface of which has second components (11) which bond specifically to the first components (12) and form a large number of measurement points (10) on a reaction carrier, with a device (6) for bringing the first components (12) in the liquid phase and the second components (11), which are coupled to the reaction carrier and bond specifically to the first components, into contact, with a device for temperature control of the measurement points, with a light source (3) for coupling in exciting light (4) for excitation of the emission of fluorescent light (7) as a function of the bonding of the first components (12) to the second components (11) of the reaction carrier (1), and with a detector (9) for recording the fluorescent light (7) emitted for determination of the bonding of the first components (12) to the second components (11) as a function of the temperature.

13. Device according to claim 12, **characterized by** optical devices for coupling the exciting light (4) into a light wave conductor, which in particular is planar.

14. Device according to claim 13, **characterized in that** the optical devices are one or more prisms (5; 5a).

15. Device according to claim 14, **characterized in that** the prism or the prisms (5; 5a) are configured to reflect the light once or several times.

16. Device according to claim 12 or 15, **characterized in that** the solid phase is made of glass or transparent plastic.

17. Device according to at least one of the preceding claims 12 to 16, **characterized by** a degassing unit, integrated into the device, for degassing the liquid phase.

18. Device according to at least one of the preceding claims 12 to 15, **characterized in that** the device for bringing the first and second components into contact can be temperature-controlled, in particular can be heated.

19. Device according to at least one of the preceding claims 12 to 16, **characterized in that** the device (6) for bringing the first components (12) into contact with the second components (11) is a flow cell, a cuvette or a sample container joined in a sealed-off manner in the region of the measurement points on the surface of the planar light wave conductor (1b) of the reaction carrier (1).

20. Device according to at least one of the preceding claims 12 to 19, **characterized in that** the reaction carrier is a biochip (1) with a planar light wave conductor on its upper side, which carries the measurement points (10).

21. Device according to at least one of the preceding claims 12 to 20, **characterized in that** the reaction carrier (1) is a glass plate which itself forms the planar light wave conductor.

22. Device according to at least one of the preceding claims 12 to 21, **characterized in that** the exciting light (4) is incident on the reaction carrier from one side of this, and **in that** fluorescent light (7) emitted by the fluorochromes bonded to the surface of the planar light wave conductor is coupled into the planar light wave conductor in the region of the evanescent field of the exciting light (4) and is guided in this and can be recorded by the recording device (8; 9) arranged on at least one end face of the planar light wave conductor (1).

23. Device according to claim 22, **characterized in that** the recording device has an imaging lens system (8) with a filter (8b) and a detector (9).

24. Device according to claim 23, **characterized in that** the detector (9) is a photomultiplier or a CCD camera.

25. Device according to at least one of the preceding claims 12 to 24, **characterized in that** a scanning device for reading the reaction carrier (1) is provided and the reaction carrier (1) and/or the exciting light from the surroundings of the reaction carrier (1) can be moved relative to this in at least one plane.

26. Device according to at least one of claims 12 to 25, **characterized in that** the device for bringing the first and second components into contact can be heated.

27. Device according to at least one of the preceding claims 12 to 26, **characterized in that** the reaction carrier carries a light wave conductor, which in particular is planar, on the surface of which are the measurement points.

28. Device according to one of claims 12 to 27, **characterized in that** a biochip (20) is pressed against a flow cell and a reaction volume (25) formed between the flow cell and biochip is sealed off by an O-ring.

29. Device according to claim 28, **characterized in that** a temperature-control device for the reaction volume is formed by a peltier element (24).

30. Device according to claim 29, **characterized in that** the peltier element is in contact with a rear side of the flow cell.

31. Device according to one of claims 28 to 30, **characterized in that** a thermally conductive metal body, in particular a copper block, heat exchange of which with the surroundings preferably influenced by a subsequent fan element (27), is joined to the peltier element.

32. Device according to one of claims 29 to 31, **characterized in that** the flow cell comprises a temperature probe, in particular a resistance thermometer, which, in connection with a regulator, in particular a PID regulator, and the peltier element, forms a control circuit.

## Revendications

1. Procédé pour la détermination en parallèle de paramètres dépendant de la température, tels que les paramètres d'association/dissociation et/ou la constante d'équilibre de complexes formés par au moins deux composants, dans lequel les premiers composants qui (12) se trouvent dans une phase liquide sont, par une mise en contact de la phase liquide et du support de réaction (1), qui a lieu en formant des complexes (13), mis en contact avec une pluralité de points de mesure (10) se trouvant sur un support de réaction, qui peut être activé par voie optique et qui est formé par des deuxièmes composants (11) qui sont couplés avec le support de réaction fixe et qui sont combinés de manière spécifique avec les premiers composants (12), dans lequel l'activation de colorants fluorescents proches de la surface supérieure, combinés avec les premiers composants (12) et/ou avec les deuxièmes composants (11), qui servent à l'émission de lumière fluorescente (7) sous l'action d'une lumière d'activation irradiée (4) et à la détection de la lumière fluorescente émise, et dans lequel la formation ou la décomposition des complexes comprenant les premiers composants (12) et les deuxièmes composants (11) est observée dans un champ de température variable en tant que fonction de la température.

2. Procédé selon l'une quelconque des revendications précédentes, **caractérisé en ce que** les premiers et/ou les deuxièmes composants sont des oligopeptides ou des polypeptides.

3. Procédé selon l'une quelconque des revendications précédentes, **caractérisé en ce que** les premiers et/ou les deuxièmes composants sont des chaînes individuelles d'acide nucléique.

4. Procédé selon l'une quelconque des revendications précédentes, **caractérisé en ce que** la lumière d'activation (4) est introduite à l'aide d'un dispositif optique, comme par exemple un ou plusieurs prismes (5 ; 5a), dans un guide, de préférence un guide d'ondes lumineuses.

5. Procédé selon l'une quelconque des revendications précédentes, **caractérisé en ce que** la lumière d'activation (4) produit, par une réflexion totale (ATR) ou par une fluorescence par réflexion interne totale (TIRF) des rayons lumineux sur une surface limite entre deux supports de densité optique différente, un champ électromagnétique dans le support optiquement le plus mince, alors que, pour la mesure de l'évolution dans le temps de la réaction, le support optiquement plus dense est une phase solide et le support optiquement plus mince est une phase liquide.

6. Procédé selon la revendication 4 ou 5, **caractérisé en ce que** sont prévus des prismes (5a) qui, à l'aide d'une réflexion multiple sur le côté supérieur et sur le côté inférieur des prismes (5a), réalisent un éclairement plan d'un champ de mesure au moyen d'une optique linéaire.

7. Procédé selon au moins l'une quelconque des revendications précédentes 1 à 6, **caractérisé en ce qu'**à l'aide d'une lumière d'activation (4) introduite dans le guide d'ondes lumineuses planaire a lieu une activation simultanée de tous les points de mesure (10).

8. Procédé selon la revendication 5, **caractérisé en ce que** la lumière fluorescente (7) des deuxièmes composants (11) combinés de manière spécifique avec les premiers composants (12) est amenée de préférence à l'aide d'une optique de reproduction comprenant un filtre (8a) à un détecteur à résolution locale (9) situé au-dessus ou en dessous de la biopuce (ou sur le côté pour la lecture de la biopuce (1)).

9. Procédé selon l'une quelconque des revendications précédentes, **caractérisé en ce que** la phase liquide fait l'objet d'un dégazage.

10. Procédé selon au moins l'une quelconque des revendications 1 à 9, **caractérisé en ce que** la lumière fluorescente est activée par le champ évanescent de la lumière d'activation, en particulier d'une lumière laser, introduite dans un guise d'ondes lumineuses planaire.

11. Procédé selon au moins l'une quelconque des revendications précédentes 1 à 9, **caractérisé en ce que** la lumière fluorescente est activée par une lumière d'activation, en particulier une lumière laser, à partir de l'environnement d'un élément optique portant les points de mesure.

12. Dispositif pour la détermination de paramètres dépendant de la température, tels que les paramètres d'association/dissociation et/ou de la constante d'équilibre de complexes formés par au moins deux composants avec un support de réaction (1), dont la surface supérieure pouvant être activée par voie optique comprend des deuxièmes composants (11) qui sont combinés de manière spécifique avec les premiers composants (12) et qui forment une pluralité de points de mesure (10) sur un support de réaction, avec un dispositif (6) pour la mise en contact des premiers composants (12) qui se trouvent dans la phase liquide avec les deuxièmes composants (11) couplés au support de réaction et combinés de manière spécifique avec les premiers composants, avec un dispositif pour régler la température des points de mesure, avec une source lumineuse (3) pour l'introduction de lumière d'activation (4) servant à activer l'émission de lumière fluorescente (7) en fonction de la combinaison des premiers composants (12) avec les deuxièmes composants (11) du support de réaction (1) et avec un détecteur (9) pour détecter la lumière fluorescente (7) émise afin de déterminer la combinaison des premiers composants (12) avec les deuxièmes composants (11) en tant que fonction de la température.

13. Dispositif selon la revendication 12, **caractérisé par** des dispositifs optiques pour introduire la lumière d'activation (4) dans un guide d'ondes lumineuses, en particulier planaire.

14. Dispositif selon la revendication 13, **caractérisé en ce que** les dispositifs optiques sont un ou plusieurs prisme(s) (5 ; 5a).

15. Dispositif selon la revendication 14, **caractérisé en ce que** le ou les prisme(s) (5 ; 5a) est(sont) conçu(s) pour assurer une réflexion simple ou multiple de la lumière.

16. Dispositif selon la revendication 12 ou 15, **caractérisé en ce que** la phase solide se compose de verre ou de matériau synthétique transparent.

17. Dispositif selon au moins l'une quelconque des revendications précédentes 12 à 16, **caractérisé par** une unité de dégazage intégrée dans le dispositif pour le dégazage de la phase liquide.

18. Dispositif selon au moins l'une quelconque des revendications précédentes 12 à 15, **caractérisé en ce que** le dispositif pour la mise en contact des premiers et des deuxièmes composants peut être réglé en température, en particulier chauffé.

19. Dispositif selon au moins l'une quelconque des revendications précédentes 12 à 16, **caractérisé en ce que** le dispositif (6) pour la mise en contact des premiers composants (12) avec les deuxièmes composants (11) est une cellule de flux, une cuvette ou un conteneur d'échantillons qui est en liaison d'étanchéité dans la zone des points de mesure sur la surface du guide d'ondes planaire (1b) du support de réaction (1).

20. Dispositif selon au moins l'une quelconque des revendications précédentes 12 à 19, **caractérisé en ce que** le support de réaction est une biopuce (1) avec un guide d'ondes planaire se trouvant sur son côté supérieur et portant les points de mesure (10).

21. Dispositif selon au moins l'une quelconque des revendications précédentes 12 à 20, **caractérisé en ce que** le support de réaction (1) est une plaque en verre qui constitue elle-même le guide d'ondes planaire.

22. Dispositif selon au moins l'une quelconque des revendications précédentes 12 à 21, **caractérisé en ce que** la lumière d'activation (4) tombe sur le support de réaction à partir d'un côté de celui-ci et **en ce que** la lumière fluorescente (7) émise par les fluorochromes combinés sur la surface du guide d'ondes planaire est introduite dans le guide d'ondes planaire dans la zone du champ évanescent de la lumière d'activation (4), est guidée dans celui-ci et peut être détectée par le dispositif de détection (8 ; 9) qui est disposé sur au moins une surface d'extrémité du guide d'ondes planaire (1).

23. Dispositif selon la revendication 22, **caractérisé en ce que** le dispositif de détection comprend une optique de reproduction (8) avec un filtre (8b) ainsi qu'avec un détecteur (9).

24. Dispositif selon la revendication 23, **caractérisé en ce que** le détecteur (9) est un photomultiplicateur ou une caméra CCD.

25. Dispositif selon au moins l'une quelconque des revendications précédentes 12 à 24, **caractérisé en ce qu'**un dispositif de balayage est prévu pour la lecture du support de réaction (1) et **en ce que** le support de réaction (1) et/ou la lumière d'activation (4) peuvent, à partir de l'environnement du support de réaction (1), être mis en mouvement par rapport à celui-ci sur au moins un plan.

26. Dispositif selon au moins l'une quelconque des revendications 12 à 25, **caractérisé en ce que** le dispositif pour la mise en contact des premiers et deuxièmes composants peut être chauffé.

27. Dispositif selon au moins l'une quelconque des revendications précédentes 12 à 26, **caractérisé en ce que** le support de réaction porte un guide d'ondes, en particulier planaire, sur la surface duquel se trouvent les points de mesure.

28. Dispositif selon l'une quelconque des revendications 12 à 27, **caractérisé en ce qu'**une biopuce (20) est pressée contre une cellule de flux et **en ce qu'**un volume de réaction (25) formé entre la cellule de flux et la biopuce est rendu étanche par un joint torique.

29. Dispositif selon la revendication 28, **caractérisé en ce qu'**un dispositif d'équilibrage des températures pour le volume de réaction est formé par un élément de Peltier (24).

30. Dispositif selon la revendication 29, **caractérisé en ce que** l'élément de Peltier est en contact avec un côté arrière de la cellule de flux.

31. Dispositif selon l'une quelconque des revendications 28 à 30, **caractérisé en ce qu'**en liaison avec l'élément de Peltier, un corps métallique thermoconducteur, en particulier un bloc en cuivre, dont l'échange de chaleur avec l'environnement est influencé de préférence par un élément de ventilateur (27) placé en aval.

32. Dispositif selon l'une quelconque des revendications 29 à 31, **caractérisé en ce que** la cellule de flux comprend un capteur de température, en particulier un thermomètre à résistance, qui, en liaison avec un régulateur, en particulier un régulateur PDI, et l'élément de Peltier, forme un circuit de régulation.
